# FASCICULE DE BREVET EUROPEEN

(11) **EP 0 876 361 B1**
(45) Date de publication et mention de la délivrance du brevet: **22.08.2001**
(21) Numéro de dépôt: 96941720.3
(22) Date de dépôt: 09.12.1996
(51) Int. Cl.: C07D 305/14, A61K 31/335

(54) **DIHYDRATE DU (2R,3S)-3-TERT-BUTOXYCARBONYLAMINO-2-HYDROXY-3-PHENYLPROPIONATE DE 4,10BETA-DIACETOXY-2ALPHA-BENZOYLOXY-5BETA,20-EPOXY-1-HYDROXY-9-OXO-19-NOR-CYCLOPROPA G]TAX-11-ENE-13ALPHA-YLE, ET SON PROCEDE DE PREPARATION**
DIHYDRAT VON (2R,3S)-3-TERT-BUTOXYCARBONYLAMINO-2-HYDROXY-3-PHENYLPROPIONSÄURE ESTER VON 4,10-BETA-DIACETOXY-2ALPHA-BENZOYLOXY-5BETA, 20-EPOXY-1-HYDROXY-9-OXO-19-NOR-CYCLOPROPA(G]TAX-11-EN-13ALPHA-YL UND VERFAHREN ZU IHRER HERSTELLUNG
4,10BETA-DIACETOXY-2ALPHA-BENZOYLOXY-5BETA,20-EPOXY-1-HYDROXY-9-OXO-19-NOR-CYCLOPROPA(G]TAX-11-ENE-13ALPHA-YL (2R,3S)-TERT-BUTOXYCARBONYLAMINO-2-HYDROXY-3-PHENYLPROPIONATE DIHYDRATE, AND METHOD FOR PREPARING SAME

(30) Priorité: 14.12.1995 FR 9514841
(43) Date de publication de la demande: 11.11.1998
(73) Titulaire: Aventis Pharma S.A., 92160 Antony (FR)
(72) Inventeur: DIDIER, Eric, F-75013 Paris (FR); LAVIGNE, Michel, F-91380 Chilly-Mazarin (FR); AUTHELIN, Jean-René, F-91180 Saint-Germain-les-Arpajon (FR)
(74) Mandataire: Le Pennec, Magali
(86) Numéro de dépôt international: FR9601957
(87) Numéro de publication internationale: WO9721695

(56) Documents cités:
- CHEMICAL ABSTRACTS, vol. 113, no. 75, 1990 Columbus, Ohio, US; abstract no. 142641k, GUERITTE-VOGELEIN ET AL.: "structure of a synthetic taxol precursor." page 723; colonne 2; XP002007111 & ACTA CRISTALLOGR. SECT. C:CRYSTAL STRUCTURE COMMUN., vol. C46, no. 5, 1990, ENGL., pages 781-784,

## Description

La présente invention concerne le dihydrate de (2R,3S)-3-tert-butoxycarbonylamino-2-hydroxy-3-phénylpropionate de 4,10β-diacétoxy-2α-benzoyloxy-5β,20-époxy-1-hydroxy-9-oxo-19-nor-cyclopropa[g]tax-11-ène-13α-yle, et sa préparation.

Le (2R,3S)-3-tert-butoxycarbonylamino-2-hydroxy-3-phénylpropionate de 4,10β-diacétoxy-2α-benzoyloxy-5β,20-époxy-1-hydroxy-9-oxo-19-norcyclopropa[g]tax-11-ène-13a-yle présente des propriétés anticancéreuses et antileucémiques remarquables.

Le (2R,3S)-3-tert-butoxycarbonylamino-2-hydroxy-3-phénylpropionate de 4,10β-diacétoxy-2α-benzoyloxy-5β,20-époxy-1-hydroxy-9-oxo-19-nor-cyclopropa[g] tax-11-ène-13α-yle est préparé selon le procédé qui est décrit plus particulièrement dans la demande internationale PCT WO 94/13654.

Il a été trouvé que le dihydrate du (2R,3S)-3-ter-butoxycarbonylamino-2-hydroxy-3-phénylpropionate de 4,10β-diacétoxy-2α-benzoyloxy-5β,20-époxy-1-hydroxy-9-oxo-19-nor-cyclopropa[g]tax-11-ène-13α-yle présente une stabilité très nettement supérieure à celle du produit anhydre.

Selon l'invention, le dihydrate du (2R,3S)-3-ter-butoxycarbonylamino-2-hydroxy-3-phénylpropionate de 4,10β-diacétoxy-2α-benzoyloxy-5β,20-époxy-1-hydroxy-9-oxo-19-nor-cyclopropa[g]tax-11-ène-13a-yle peut être obtenu par cristallisation du (2R,3S)-3-tert-butoxycarbonylamino-2-hydroxy-3-phénylpropionate de 4,10β-diacétoxy-2α-benzoyloxy-5β,20-époxy-1-hydroxy-9-oxo-19-nor-cyclopropa [g]tax-11-ène-13α-yle ou par recristallisation du dihydrate dans un mélange d'eau et d'un alcool aliphatique contenant 1 à 3 atomes de carbone, ou dans un mélange d'eau et d'une cétone contenant 3 à 4 atomes de carbone suivi du séchage sous pression réduite du produit isolé et d'un maintien éventuel dans une humidité relative supérieure ou égale à 40 % ou d'un séchage direct dans une atmosphère dont l'humidité relative est supérieure ou égale à 40 %

Pour la mise en oeuvre du procédé selon l'invention, il peut être particulièrement avantageux
- de mettre en solution le (2R,3S)-3-tert-butoxycarbonylamino-2-hydroxy-3-phénylpropionate de 4,10β-diacétoxy-2α-benzoyloxy-5β,20-époxy-1-hydroxy-9-oxo-19-nor-cyclopropa[g]tax-11-ène-13α-yle ou son hydrate dans un alcool aliphatique contenant 1 à 3 atomes de carbone, ou dans une cétone contenant 3 à 4 atomes de carbones,
- de traiter la solution par de l'eau,
- d'ensemencer la solution avec le dihydrate puis de traiter de nouveau à l'eau,
- de séparer les cristaux obtenus, puis
- de les sécher sous pression réduite puis de les maintenir éventuellement dans une atmosphère dont l'humidité relative est supérieure ou égale à 40 %, c'est-à-dire, à titre d'exemple pour une humidité relative de 40 %, un séchage sous une pression résiduelle d'environ 1,33 kPa pour une température de 25°C et sous une pression résiduelle d'environ 3.86 kPa pour une température de 45°C.
- ou de les sécher directement dans une atmosphère dont l'humidité relative est supérieure ou égale à 40 %, c'est-à-dire, à titre d'exemple pour une humidité relative de 40 %, un séchage sous une pression résiduelle d'environ 1,33 kPa pour une température de 25°C et sous une pression résiduelle d'environ 3,86 kPa pour une température de 45°C.

Généralement, le (2R,3S)-3-tert-butoxycarbonylamino-2-hydroxy-3-phénylpropionate de 4,10β-diacétoxy-2α-benzoyloxy-5β,20-époxy-1-hydroxy-9-oxo-19-norcyclopropa[g]tax-11-ène-13α-yle ou son hydrate est dissous dans un excès de l'alcool aliphatique, de préference l'éthanol ou dans un excés de la cétone, de préférence l'acétone. De préférence, la quantité d'alcool ou de cétone est comprise entre 4 et 16 parties en volume par rapport au poids de (2R,3S)-3-tert-butoxycarbonylamino-2-hydroxy-3-phénylpropionate de 4,10β-diacétoxy-2α-benzoyl-oxy-5β,20-époxy-1-hydroxy-9-oxo-19-nor-cyclopropa[g]tax-11-ène-13α-yle mis en oeuvre.

Généralement, l'eau est ajoutée de telle façon que le rapport de volume final eau/alcool ou eau/cétone soit compris entre 1/3 et 3/1.

Le dihydrate de (2R,3S)-3-tert-butoxycarbonylamino-2-hydroxy-3-phénylpropionate de 4,10β-diacétoxy-2α-benzoyloxy-5β,20-époxy-1-hydroxy-9-oxo-19-norcyclopropa[g]tax-11-ène-13α-yle qui cristallise est séparé, de préférence par filtration ou centrifugation. Le séchage ou le maintien éventuel dans une atmosphère dont l'humidité relative est supérieure ou égale à 40 % est effectué sous pression réduite, comprise généralement entre 0,5 et 30 kPa, de préférence voisine de 5 kPa à une température comprise entre 10 et 70°C, de préférence voisine de 40°C .

L'isotherme d'activité en eau du produit a été étudié. Ainsi des échantillons de (2R,3S)-3-tert-butoxycarbonylamino-2-hydroxy-3-phénylpropionate de 4,10β-diacétoxy-2α-benzoyloxy-5β,20-époxy-1-hydroxy-9-oxo-19-nor-cyclopropa[g]tax-11-ène-13α-yle dihydrate volontairement déshydratés par séchage poussé ont été maintenu dans des atmosphères d'humidité relative (HR) contrôlée à 25°C. Les teneurs en eau, déterminées par ATG montrent que les produits se stabilisent à des teneurs proches de 4 % (entre 3 et 5 %) lorsque l'humidité relative est supérieure ou égale à 40 % (teneur en eau théorique de 4,15 % pour un dihydrate).

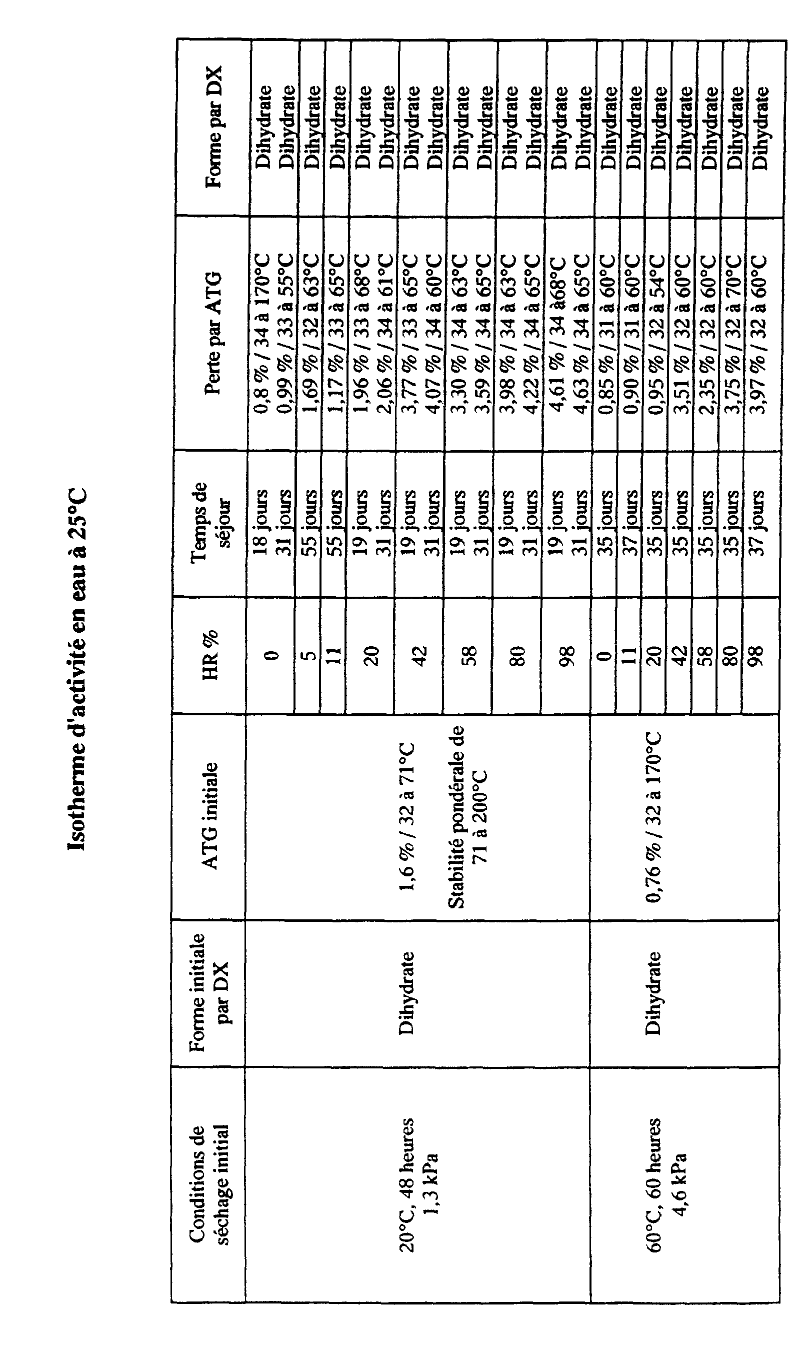

Pour la mise en oeuvre du procédé selon l'invention, il est possible d'opérer directement sur la solution éthanolique du (2R,3S)-3-tert-butoxycarbonylamino-2-hydroxy-3 -phénylpropionate de 4,10β-diacétoxy-2α-benzoyloxy-5β,20-époxy-1-hydroxy-9-oxo-19-nor-cyclopropa[g]tax-11-ène-13α-yle obtenu après déprotection en milieu acide du (2R,4S,5R)-3-tert-butoxycarbonyl-2-(4-méthoxyphényl)-4-phényloxazolidine-5-carboxylate de 4,10β-diacétoxy-2α-benzoyloxy-5β,20-époxy-1-hydroxy-9-oxo-19-nor-cyclopropa [g] tax-11-ène-13α-yle.

Les exemples suivants illustrent la présente invention.

### EXEMPLE 1

Dans un tricol, on introduit 2g de (2R,3S)-3-tert-butoxycarbonylamino-2-hydroxy-3-phénylpropionate de 4,10β-diacétoxy-2α-benzoyloxy-5β,20-époxy-1-hydroxy-9-oxo-19-nor-cyclopropa[g]tax-11-éne-13α-yle brut et 20 cm3 d'éthanol absolu. A la solution agitée à 50°C, on additionne en 45 minutes environ 20 cm3 d'eau puis la suspension obtenue est refroidie à une température voisine de 20°C. Après filtration, le produit est lavé sur le filtre avec 20 cm3 d'un mélange éthanol absolu-eau (1-1 en volumes), puis le produit est séché à 40°C sous pression réduite (5,3 kPa). On obtient 1,64 g du dihydrate de (2R,3S)-3-tert-butoxycarbonylamino-2-hydroxy-3-phénylpropionate de 4,10β-diacétoxy-2α-benzoyloxy-5β,20-époxy-1-hydroxy-9-oxo-19-nor-cyclopropa[g]tax-11-ène-13α-yle contenant 3,8 % par ATG d'eau (valeur théorique de la teneur en eau du dihydrate de 4,15 %). Le rendement obtenu est d'environ 80 %. Le diagramme DPRX (diagramme de poudre aux rayons X montre que le produit ainsi obtenu présente les caractéristiques du dihydrate.

Le diagramme de poudre aux rayons X est réalisé au moyen d'un appareil Philips PW 1700® à tube anti-cathode de cobalt (λ K_{α1} = 1,7889 Å), le balayage étant effectué sous un angle de balayage initial de 5° 2-θ, de balayage final de 40° 2-θ, avec un pas de 0,02° 2-θ, à raison de 1 seconde par pas et en utilisant une pastille de silicium.

L'analyse thermogravimétrique (ATG) est effectuée au moyen d'une thermobalance Perkin-Elmer TGA 7® à la température initiale de 25°C et à la température finale de 300°C avec un gradient de température de 10°C par minute.

### EXEMPLE 2

Dans un réacteur purgé à l'azote, on introduit 515 g du dihydrate de (2R,3S)-3-ter-butoxycarbonylamino-2-hydroxy-3-phénylpropionate de 4,10β-diacétoxy-2α-benzoyloxy-5β,20-époxy-1-hydroxy-9-oxo-9-nor-cyclopropa[g]tax-11-éne-13α-yle brut et 4 litres d'éthanol absolu. On chauffe la suspension à 35-40°C jusqu'à dissolution et le réacteur est mis sous une pression réduite de 8 kPa. On distille environ 8 litres de solvant à volume constant en alimentant en continu le réacteur avec de l'éthanol (8 litres).Après remise du réacteur à la pression atmosphérique, la solution est clarifiée par passsage sur un filtre equipée d'une toile filtrante de 0,45 µm. Après rinçage du filtre avec 1 litre d'éthanol absolu, on additionne en 1 heure à 40°C sur la totalité de la solution environ 1,8 litre d'eau. Après amorçage avec 8 g du dihydrate de (2R,3S)-3-tert-butoxycarbonylamino-2-hydroxy-3-phénylpropionate de 4,10β-diacétoxy-2α-benzoyloxy-5β,20-époxy-1-hydroxy-9-oxo-19-norcyclopropa[g]tax-11-ène-13α-yle, le mélange est refroidi à température voisine de 20°C pendant 15 heures. La suspension est alors chauffée à 40°C puis on ajoute en 4 heures 1,66 litre d'eau. Le mélange est refroidi à une température voisine de 20°C et laissé sous agitation pendant 17 heures. La suspension est filtrée sur verre fritté et le produit essoré est lavé avec 1,25 litre d'un mélange alcool-eau (50-50 en volumes). Le produit est séché dans une étuve à 35°C sous 5,3 kPa pendant 72 heures en présence d'une réseve d'eau puis à 35°C sous 2,7 kPa pendant 8 heures sans réserve d'eau et de nouveau à 35°C sous 5,3 kPa pendant 16 heures en présence d'une réserve d'eau. On obtient ainsi 491 g du dihydrate de (2R,3S)-3-tert-butoxycarbonylamino-2-hydroxy-3-phénylpropionate de 4,10β-diacétoxy-2α-benzoyloxy-5β,20-époxy-1-hydroxy-9-oxo-l9-nor-cyclopropa[g] tax-11-ène-13α-yle contenant 4,0 % d'eau (Karl Fischer). Le diagramme DPRX (diagramme de poudre aux rayons X) montre que le produit ainsi obtenu se présente sous forme d'un dihydrate (valeur théorique de la teneur en eau du dihydrate de (2R.3S)-3-tert-butoxycarbonylamino-2-hydroxy-3-phényl-propionate de 4,10β-diacétoxy-2α-benzoyloxy-5β,20-époxy-1-hydroxy-9-oxo-19-nor-cyclopropa[g]tax-11-ène-13α-yle de 4,15 %).

Le diagramme de poudre aux rayons X est réalisé dans les conditions décrites dans l'exemple 1.

### EXEMPLE 3

Dans un ballon tricol, on introduit 2 g de (2R,3S)-3-tert-butoxycarbonylamino-2-hydroxy-3-phénylpropionate de 4,10β-diacétoxy-2α-benzoyloxy-5β,20-époxy-1-hydroxy-9-oxo-19-nor-cyclopropa[g]tax-11-ène-13α-yle brut et 20 cm3 d'acétone. A la solution obtenue qui est agitée à une température voisine de 20°C, on ajoute en 35 minutes environ 20 cm3 d'eau. La suspension obtenue est agitée pendant 15 minutes environ. Après filtration, le produit est lavé deux fois sur le filtre avec 20 cm3 d'un mélange acétone-eau (1-1 en volumes), puis le produit est séché à 40°C sous pression réduite (5.3 kPa). On obtient ainsi 1,28 g du dihydrate de (2R,3S)-3-tert-butoxycarbonylamino-2-hydroxy-3-phénylpropionate de 4,10β-diacétoxy-2α-benzoyloxy-5β,20-époxy-1-hydroxy-9-oxo-19-nor-cyclopropa[g]tax-11-ène-13α-yle. Le rendement obtenu est d'environ 75 %. Le diagramme DPRX (diagramme de poudre aux rayons X) montre que le produit ainsi obtenu présente les caractéristiques du dihydrate.

Le diagramme de poudre aux rayons X est réalisé dans les conditions décrites dans l'exemple 1.

### EXEMPLE 4

Dans un réacteur purgé à l'azote, on introduit 120 g de (2R,4S,5R)-3-tert-butoxycarbonyl-2-(4-méthoxyphényl)-4-phényloxazolidine-5-carboxylate de 4,10β-diacétoxy-2α-benzoyloxy-5β,20-époxy-1-hydroxy-9-oxo-19-nor-cyclopropa[g]tax-11-ène-13α-yle et 1,08 litre d'acétate d'éthyle. La suspension est agitée à une température voisine de 20°C, puis on introduit une solution de 3,25 cm3 d'acide chlorhydrique à 36 % dans 17,6 cm3 d'eau. Après maintien pendant 3 heures 30 minutes, on charge dans le réacteur une solution de 3,5 g d'hydrogénocarbonate de sodium dans 350 cm3 d'eau et on agite pendant 15 minutes. Après décantation et soutirage de la phase aqueuse, on lave deux fois de suite avec 350 cm3 d'eau.La phase organique est concentrée sous pression réduite à environ 25°C jusqu'à un volume résiduel de 350 cm3 puis on introduit 350 cm3 d'éthanol absolu. La distillation sous pression réduite est reprise à environ 30°C jusqu'à épuisement de l'acétate d'éthyle en alimentant avec 1,5 litre d'éthanol absolu. La solution est chauffée à 40°C puis on ajoute en 15 minutes 470 cm3 d'eau. La solution est ensemencée avec une suspension de 1g du dihydrate de (2R,3S)-3-tert-butoxycarbonylamino-2-hydroxy-3-phényl-propionate de 4,10β-diacétoxy-2α-benzoyloxy-5β,20-époxy-1-hydroxy-9-oxo-19-nor-cyclopropa[g]tax-11-ène-13α-yle dans un mélange de 40 cm3 d'eau et 40 cm3 d'éthanol absolu. Après maintien pendant 15 heures à environ 40°C, on ajoute en 4-5 heures 410 cm3 d'eau puis on refroidit la suspension à 20°C. Après filtration et lavage du produit sur filtre, le produit est séché pendant 15 heures sous pression réduite (2,7 kPa) à 25°C puis pendant 24 heures à 35°C.On obtient ainsi 102g du dihydrate de (2R,3S)-3-tert-butoxycarbonylamino-2-hydroxy-3-phénylpropionate de 4,10β-diacétoxy-2α-benzoyloxy-5β,20-époxy-1-hydroxy-9-oxo-19-nor-cyclopropa[g]tax-11-ène-13α-yle. contenant 4,3 % d'eau (Karl Fischer), avec un rendement d'environ 93 %. Le diagramme DPRX (diagramme de poudre aux rayons X) montre que le produit ainsi obtenu présente les caractéristiques du dihydrate.

Le diagramme de poudre aux rayons X est réalisé dans les conditions décrites dans l'exemple 1.

## Revendications

1. Le dihydrate de (2R,3S)-3-ter-butoxycarbonylamino-2-hydroxy-3-phénylpropionate de 4,10β-diacétoxy-2α-benzoyloxy-5β,20-époxy-1-hydroxy-9-oxo-19-nor-cyclopropa[g]tax-11-ène-13α-yle.

2. Procédé de préparation du dihydrate de (2R,3S)-3-tert-butoxycarbonylamino-2-hydroxy-3-phénylpropionate de 4,10β-diacétoxy-2α-benzoyloxy-5β,20-époxy-1-hydroxy-9-oxo-19-nor-cyclopropa[g]tax-11-ène-13α-yle caractérisé en ce que l'on cristallise le (2R,3S)-3-tert-butoxycarbonylamino-2-hydroxy-3-phénylpropionate de 4,10β-diacétoxy-2α-benzoyloxy-5β,20-époxy-1-hydroxy-9-oxo-19-nor-cyclopropa[g]tax-11-ène-13α-yle ou son hydrate dans un mélange d'eau et d'un alcool aliphatique contenant 1 à 3 atomes de carbone ou dans un mélange d'eau et d'une cétone contenant 3 à 4 atomes de carbone, que l'on sèche le produit obtenu sous pression réduite et qu'on le maintient éventuellement dans des conditions d'humidité relative supérieures ou égales à 40 %, ou que l'on sèche le produit directement dans des conditions d'humidité relative supérieures ou égales à 40 %.

3. Procédé selon la revendication 2 caractérisé en ce que le rapport volumique final eau/alcool et eau/cétone est compris entre 3/1 à 1/3.

4. Procédé selon l'une des revendications 2 ou 3 caractérisé en ce que l'alcool est l'éthanol et la cétone est l'acétone.

5. Procédé selon la revendication 2 caractérisé en ce que le séchage ou le maintien éventuel sous une humidité relative supérieure ou égale à 40 % est effectué à une température voisine de 40°C sous une pression voisine de 5 kPa et que le produit se stabilise dans ces conditions entre 3 et 5 % d'eau.

6. Procédé selon la revendication 2 caractérisé en ce que l'on opère directement à partir de la solution éthanolique du (2R,3S)-3-tert-butoxycarbonylamino-2-hydroxy-3-phénylpropionate de 4,10β-diacétoxy-2α-benzoyl- oxy-5β,20-époxy-1-hydroxy- 9-oxo-19-nor-cyclopropa[g]tax-11-ène-13α-yle obtenu par déprotection en milieu acide de l'ester (2R,4S,5R)-3-tert-butoxycarbonyl-2-(4-méthoxyphényl)-4-phényloxazolidine-5-carboxylate de 4,10β-diacétoxy-2α-benzoyloxy-5β,20-époxy-1-hydroxy-9-oxo-19-nor-cyclopropa[g]tax-11-ène-13α-yle.

## Claims

1. 4,10β-Diacetoxy-2α-benzoyloxy-5β,20-epoxy-1-hydroxy-9-oxo-19-norcyclopropa[g]tax-11-en-13α-yl (2R,3S)-3-tert-butoxycarbonylamino-2-hydroxy-3-phenylpropionate dihydrate.

2. Process for the preparation of 4,10β-diacetoxy-2α-benzoyloxy-5β,20-epoxy-1-hydroxy-9-oxo-19-norcyclopropa[g]tax-11-en-13α-yl (2R,3S)-3-tert-butoxycarbonylamino-2-hydroxy-3-phenylpropionate dihydrate, characterized in that 4,10β-diacetoxy-2α-benzoyloxy-5β,20-epoxy-1-hydroxy-9-oxo-19-norcyclopropa[g]tax-11-en-13α-yl (2R,3S)-3-tert-butoxycarbonylamino-2-hydroxy-3-phenylpropionate or its hydrate is crystallized from a mixture of water and an aliphatic alcohol containing 1 to 3 carbon atoms or from a mixture of water and a ketone containing 3 to 4 carbon atoms, in that the product obtained is dried under reduced pressure and in that it is optionally maintained under relative humidity conditions greater than or equal to 40 %, or in that the product is dried directly under relative humidity conditions greater than or equal to 40 %.

3. Process according to Claim 2, characterized in that the water/alcohol and water/ketone final ratio by volume is between 3/1 to 1/3.

4. Process according to either of Claims 2 or 3, characterized in that the alcohol is ethanol and the ketone is acetone.

5. Process according to Claim 2, characterized in that the drying or the optional maintaining under a relative humidity greater than or equal to 40 % is carried out at a temperature in the region of 40°C under a pressure in the region of 5kPa and that the product stabilizes under these conditions at between 3 and 5% of water.

6. Process according to Claim 2, characterized in that the preparation is carried out directly from the ethanolic solution of 4,10β-diacetoxy-2α-benzoyloxy-5β,20-epoxy-1-hydroxy-9-oxo-19-norcyclopropa[g]tax-11-en-13α-yl (2R,3S)-3-tert-butoxycarbonylamino-2-hydroxy-3-phenylpropionate obtained by deprotection in acid medium of the ester 4,10β-diacetoxy-2α-benzoyloxy-5β,20-epoxy-1-hydroxy-9-oxo-19-norcyclopropa[g]tax-11-en-13α-yl (2R,4S,5R)-3-tert-butoxycarbonyl-2-(4-methoxyphenyl)-4-phenyloxazolidine-5-carboxylate.

## Patentansprüche

1. 4,10β-Diacetoxy-2α-benzoyloxy-5β,20-epoxy-1-hydroxy-9-oxo-19-nor-cyclopropa[g]tax-11-en-13α-yl-(2R, 3S)-3-tert.-butoxycarbonylamino-2-hydroxy-3-phenylpropionatdihydrat.

2. Verfahren zur Herstellung von 4,10β-Diacetoxy-2α-benzoyloxy-5β,20-epoxy-1-hydroxy-9-oxo-19-nor-cyclopropa[g]tax-11-en-13α-yl-(2R, 3S)-3-tert.-butoxycarbonylamino-2-hydroxy-3-phenylpropionat-dihydrat, dadurch gekennzeichnet, daß man 4,10β-Diacetoxy-2α-benzoyloxy-5β,20-epoxy-1-hydroxy-9-oxo-19-nor-cyclopropa[g]tax-11-en-13α-yl-(2R, 3S)-3-tert.-butoxycarbonylamino-2-hydroxy-3-phenylpropionat oder sein Hydrat in einem Gemisch von Wasser und einem aliphatischen Alkohol mit 1 bis 3 Kohlenstoffatomen oder in einem Gemisch von Wasser und einem Keton mit 3 bis 4 Kohlenstoffatomen kristallisiert, das erhaltene Produkt unter vermindertem Druck trocknet und es gegebenenfalls unter Bedingungen einer relativen Feuchtigkeit von höher als oder gleich 40% hält oder das Produkt direkt unter Bedingungen einer relativen Feuchtigkeit von höher als oder gleich 40% trocknet.

3. Verfahren gemäß Anspruch 2, dadurch gekennzeichnet, daß das endgültige Volumenverhältnis Wasser/Alkohol und Wasser/Keton zwischen 3:1 und 1:3 liegt.

4. Verfahren gemäß einem der Ansprüche 2 oder 3, dadurch gekennzeichnet, daß der Alkohol Ethanol ist und das Keton Aceton ist.

5. Verfahren gemäß Anspruch 2, dadurch gekennzeichnet, daß die Trocknung oder das eventuelle Belassen unter einer relativen Feuchtigkeit von höher als oder gleich 40% bei einer Temperatur um 40°C und unter einem Druck um 5 kPa erfolgt und daß das Produkt sich unter diesen Bedingungen zwischen 3 und 5% Wasser stabilisiert.

6. Verfahren gemäß Anspruch 2, dadurch gekennzeichnet, daß man direkt ausgeht von einer ethanolischen Lösung des 4,10β-Diacetoxy-2α-benzoyloxy-5β,20-epoxy-1-hydroxy-9-oxo-19-nor-cyclopropa[g]tax-11-en-13α-yl-(2R, 3S)-3-tert.-butoxycarbonylamino-2-hydroxy-3-phenylpropionats, das erhalten wird durch Schutzgruppenentfernung in saurem Milieu an dem Ester 4,10β-Diacetoxy-2α-benzoyloxy-5β,20-epoxy-1-hydroxy-9-oxo-19-nor-cyclopropa[g]tax-11-en-13α-yl-(2R, 4S, 5R)-3-tert.-butoxycarbonyl-2-(4-methoxyphenyl-4-phenyloxazolidin-5-carboxylat.
